# EUROPEAN PATENT SPECIFICATION

(11) **EP 3 990 230 B1**
(45) Date of publication and mention of the grant of the patent: **30.04.2025**
(21) Application number: 20832205.7
(22) Date of filing: 24.06.2020
(51) Int. Cl.: B25J 9/00

(54) **WEARABLE STRENGTH-SUPPORTING SYSTEM FOR UPPER LIMBS**
TRAGBARES KRAFTUNTERSTÜTZENDES SYSTEM FÜR DIE OBEREN GLIEDMASSEN
SYSTÈME DE SUPPORT DE FORCE PORTABLE DESTINÉ AUX MEMBRES SUPÉRIEURS

(30) Priority: 25.06.2019 TR 201909434
(43) Date of publication of application: 04.05.2022
(73) Proprietor: TB Teknoloji Sanayi Ve Ticaret Anonim Sirketi, Eskisehir (TR)
(72) Inventor: BURSOY, Gokhan, Odunpazan /Eskisehir (TR)
(74) Representative: Zaboliene, Reda
(86) International application number: PCT/TR2020/050546
(87) International publication number: WO 2020/263209

(56) References cited:
- WO-A1-2018/218336
- CN-A- 108 789 373
- CN-A- 109 605 329
- FR-A1- 3 065 387
- GB-A- 2 519 323
- US-A1- 2012 184 880
- US-A1- 2014 033 391
- MRTESLONIAN: "Exoskeleton, Wearing and testing the upper body's lifting capability", 29 August 2015 (2015-08-29), pages 1 - 1, XP054981465, Retrieved from the Internet <URL:https://www.youtube.com/watch?v=9H_s-18aeNo>

## Description

### Technical Field

The present invention relates to a wearable strength-supporting system which provides support to the arms of the user by creating a controlled balancing force to the carried load while a weight is lifted by the user.

### State of the Art

It is possible to use the wearable strength-supporting system also known as the exoskeleton to use in many fields such as military, industry, agriculture and transportation etc. These systems provide a healthy working opportunity beyond the boundaries of the human body in terms of efficiency and strength by aiming to reduce the loads coming to the muscle and skeleton system in cases where the user must carry heavy loads for long periods or in a repetitive manner.

The exoskeletons are used in the medical field for the purpose of physical rehabilitation.

A system which is an example of the wearable strength-supporting systems, meets the force coming from the arm of the user and follows the arm movement, meets the loads based on gravity partially is patented (M. Doyle, "ADAPTIVE ARM SUPPORT SYSTEMS AND METHODS FOR USE", VVO/2012/099995).

Eksovest product is an exoskeleton which is developed by Ekso Bionics Company, increases the capacity, strength and power of the user in the works which is performed with stretched arms. There is no power unit in this system and said support is enabled by means of preloaded mechanical components.

The patent file (FR 3 065 387) describes a collaborative passive energy exoskeleton designed to assist workers in handling tools and loads. It utilizes innovative techniques such as isoelastic and anisoelastic mechanical arms, an interface for user interaction, and various harness architectures. Additionally, it incorporates pantograph constructions for the exoarms, enabling their extension and retraction in response to the user's movements, thus enhancing adaptability and usability.

### Aim of the Invention

In current systems, the systems which are defined as active (having power supply) exoskeleton and use a power source and electric motor are both very heavy and lead to high costs. The invention aims at providing a system that is both lightweight and low cost, with the use of components which are few in number and light weight.

Accordingly, the present invention provides a system as defined in appended claim 1.

The system is adjusted for a load value and support is provided only at this load rate in applications where motor is not used. When the user does not carry any load, this condition creates a resistance against the movement of the user. This makes it impossible for the user practically to work without any load or with different weights. In the active (having power source) systems that use motor, the size of the load to be lifted by the user is detected by the system and thus the balancing counter force to be applied by the system is determined according to this measurement, therefore it will be possible for the user to work with different weights without making any adjustment. The instant compulsions of the motor lead to instant impacts on the muscular and skeletal system of the user in the applications where the motor is used. At the same time, the load measurement in these systems shall be performed with high precision and by considering the position of the load (height, distance to the motor etc.).

The strength control system, gas piston and strength measurement system which are among the components of the invention can both perform measurement with high precision and they do not transfer the impacts encountered during instant stresses to the uses. In order to solve a problem in the energy source or to meet a demand of the user, a version of the invention that does not use motor and does not have a power source is also the subject of the invention. The size of the support strength can be easily adjusted with an adjustment arm in this system.

### Description of the Figures

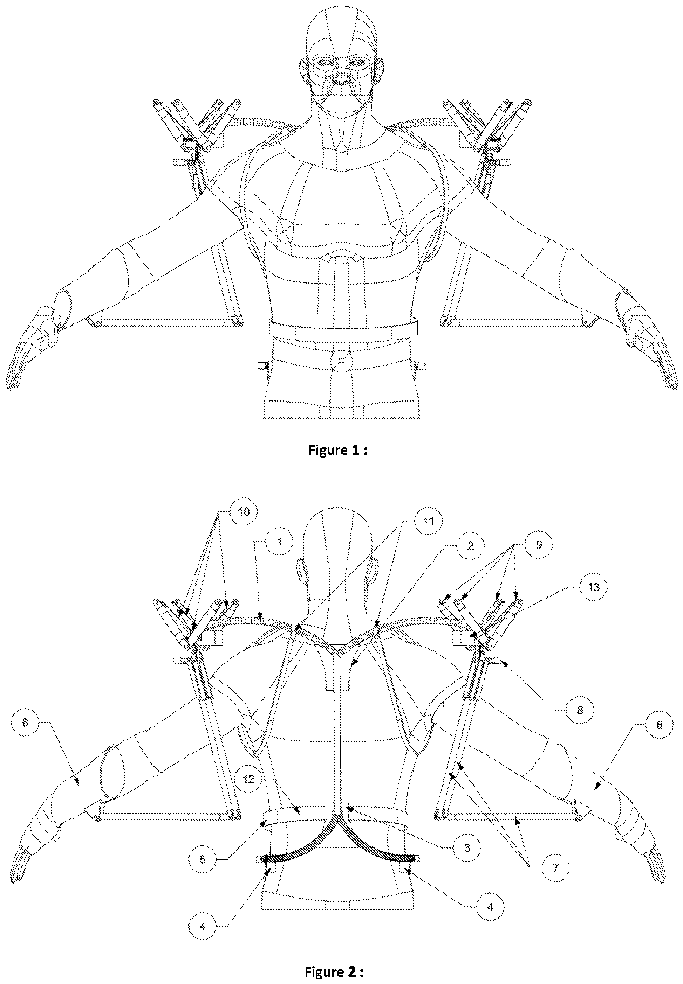

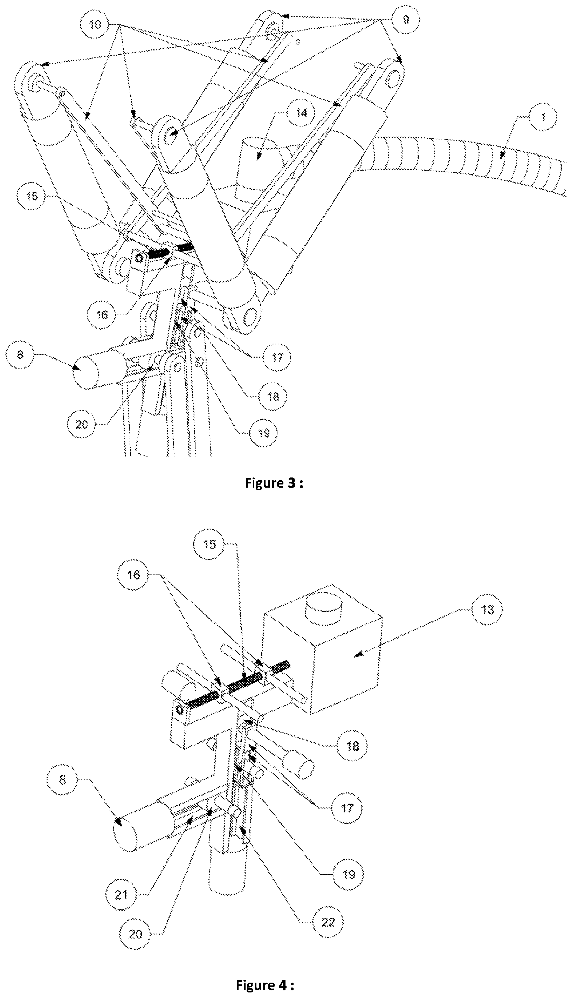

Figure 1: Front View of the Wearable Strength-Supporting system in a worn manner
Figure 2: Rear View of the Wearable Strength-Supporting system in a worn manner
Figure-3: Close-up view of the Strength Control System and the Carrier Slot
Figure 4: Carrier Slot, Horizontal and Vertical Slide and Beds, Electric Motor and Ball Screw

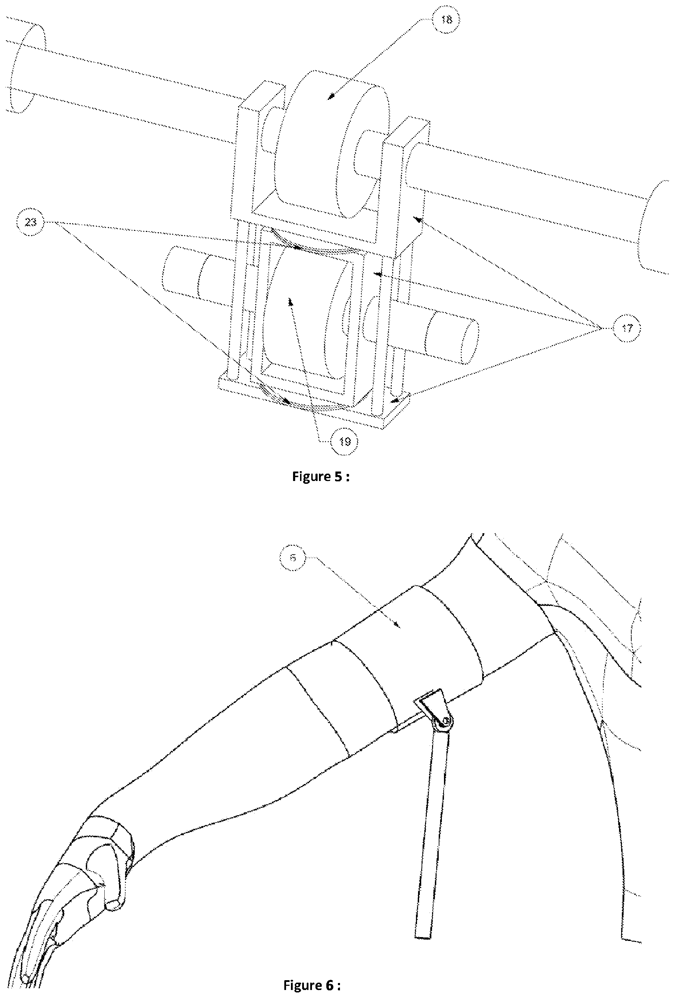

Figure 5: Close-up View of Sensor Slot, Sensors and Vertical Bed and Slides
Figure 6: Biceps support

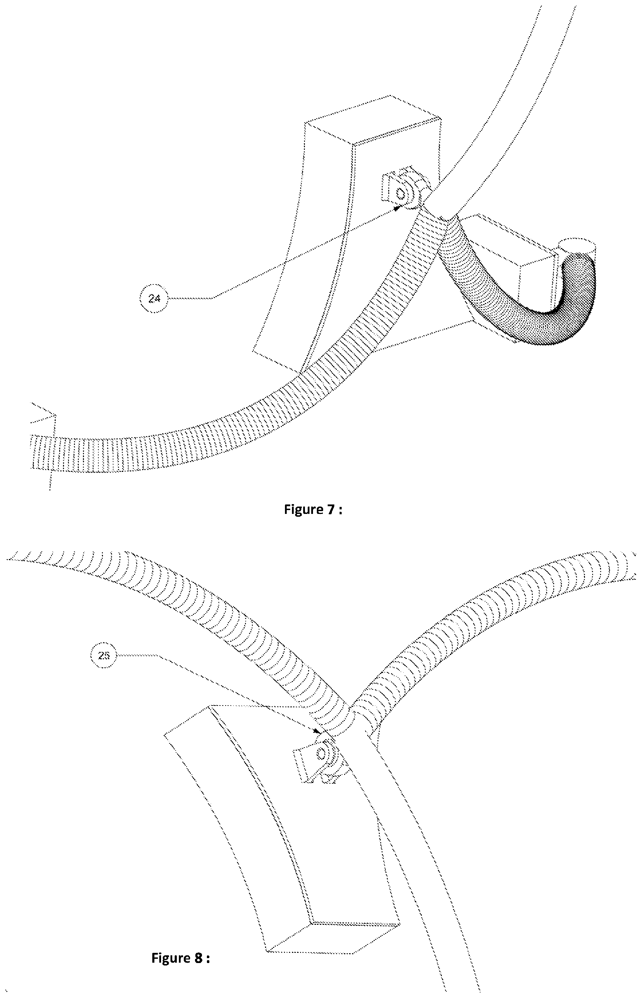

Figure 7: Waist Support Rotary Joint
Figure 8: Back Support Rotary Joint

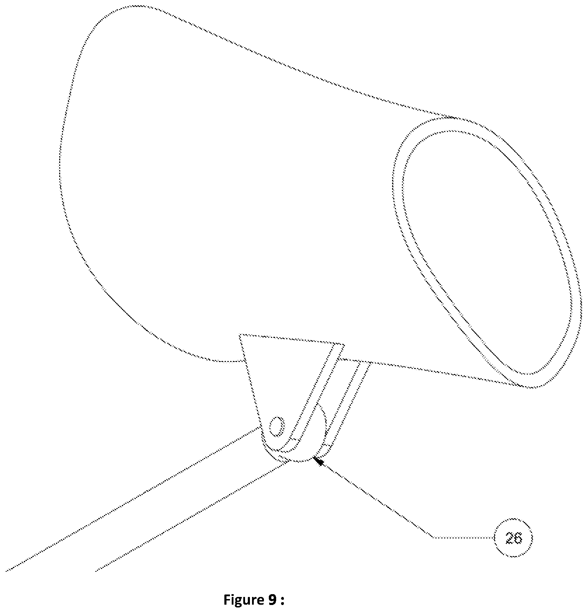

Figure 9: Arm Support Rotary Joint

### Description of the References in the Figures

- 1: : Body skeleton
- 2: : Back support
- 3: : Waist support
- 4: : Side hip supports
- 5: : Waist Belt
- 6: : Wrist and Biceps support
- 7: : Carrier Arms
- 8: : Carrier Slot
- 9: : Gas Pistons
- 10: : Brackets
- 11: : Shoulder Strap
- 12: : Waist Belt
- 13: : Electric motor
- 14: : Planar Rotary Joint between Body Skeleton-Carrier Slot
- 15: : Ball Screw
- 16: : Reverse Gear Bearings
- 17: : Sensor Housing
- 18: : Top Vertical Bed
- 19: : Bottom Vertical Bed
- 20: : Horizontal Bed
- 21: : Horizontal Slide
- 22: : Vertical Slide
- 23: : Force Sensors
- 24: : Waist Support Rotary Joint
- 25: : Back Support Rotary Joint
- 26: : Arm Support Rotary Joint

### Description of the Invention

The invention is a wearable Strength Supporting System, consisting of three main components, namely the body skeleton (1), carrier arms (7) and strength control system. The main features of the system are as follows:
- it is fixed to the human body from waist (3), back (2) and shoulder (11) regions and arms (6),
- it has carrier arms (7) which form a respective planar pantograph structure for each of the right and left arms, are connected with planar rotary joints having low friction and have metallic or composite structure,
- it has a strength control system which allows the carried load to be moved to the required height without applying any force in the vertical direction such that the object does not have any weight and forms a balancing counter force by detecting the weight of the carried load.

The parts of the system that will contact the human body are manufactured from the leather or fabric, on the waist, back, side hips and wrists or biceps as the main contact points, there are waist support (3), back support (2), side hip support (4) and wrist or biceps supports (6) which are filled with sponge or similar soft and flexible structure to reduce pressure on the body, have an outer part made of hard plastic, metal or composite. There is a waist belt (5) with adjustable height on the abdominal region that is one of the points that carry the force reactions of the moments formed in the system loading condition, which is connected to the waist support and enables force transfer to the front portion of the body.

There is a body skeleton (1) which connects the waist, back and side hip contact points, comprises side hip contact points and is made of a metallic or composite material. This skeleton (1) has rotary joints (24, 25) which enable to transfer the force to the body by rotating at the contact points of the body in the waist (3) and back (2) region.

The shoulder straps (11) fixed on the body skeleton (1) provides fixing on both shoulders in order to transfer the weight of the system in the vertical direction to the body. The height of the shoulder straps (11) can be adjustable.

The body skeleton (1) also comprises planar rotary joints (14), one for right arm and one for left arm which allows the whole system to rotate on the vertical axis, enables the connection of the carrier slot (8) where the horizontal and vertical movement of the carrier arms (7) are controlled, to the body skeleton (1).

The carrier slots (8) are components which control the horizontal and vertical movement of the carrier arms (7) and enable the connection of the carrier arms (7) to the body skeleton (1). Horizontal and vertical movement controlled on this housing provides bearings (18, 19, 20) and slides (21, 22) that allow linear motion with low friction.

The movement of the slide (21) which is aligned horizontally and bearing (20) is free as much as allowed by the size of the slide. This slide (21) and the bearing (20) enable the user to move the load carried in the horizontal axis without being exposed to any opposing resistance other than friction.

The slides (21) which are aligned in vertical direction are the components with low friction which provides the user to move the carried load in the vertical direction without applying any force to a required height as if the object does not have any weight. This section consists of two separate beds (18, 19). These two horizontals (18,19) are connected by the sensor slot (17) to each other.

There are brackets (10) in the system which are positioned crosswise and are supported with each other. One end of these brackets (10) is connected to the reverse gear bearings (16) on the ball screw (15) and the other end is connected to the gas pistons (9) by means of planar rotary joints. These brackets (9) are the mechanical components which enable to drive the ball screw (15) connected to the electric motor (13) and thus enable angular positioning of the gas pistons (9) by means of the bearings (16) that move back and forth.

The gas pistons (9), forming the mechanical base of the strength support, provide a force as much as the preloading independent of the piston extension continuously. The strength support in the invention is provided by the preloads of the gas pistons (9). The counter movement of the movement of user arm in the vertical direction in the carrier housing (8) is met by the opening and closing of the gas pistons (9). One end of these pistons (9) is connected to one of the vertical bearings (18) on the carrier housing (8) and the other end is connected to the brackets (10) by means of planar rotary joints. In case the movement of the brackets (10) is realized such that they rotate moving away from the vertical axis, the support forces applied to the system in the vertical direction reduce vectorially, because they give the same force towards each other in the horizontal direction, they are positioned such that they do not create further resistance in this direction.

The carrier arms (7), one for each arm of the user, have metallic or composite structures with a constant moment arm ratio. The carrier arms (7) are connected with planar rotary joints to each other which works with pantograph structure on the same plane, have low friction.

There is an arm support (6) at the contact point to the arm of the user between the biceps and wrist (6) which reduces the pressure to the arm such that it has a filling from sponge or similar soft and flexible structure in the internal part, has a rigid structure on the external part. The end point of the carrier arms (7) are connected to the rigid outer surface by means of a rotary joint (26). This connection (26) allows the user to move his/her arm in a rotational manner at the biceps and wrist points (6).

The strength control system is the component which positions the brackets (10) by determining the angular position of the gas pistons (9) in order to provide the required balancing force to the bearing that works in the vertical direction such that the user is able to move the load carried in the vertical direction without applying any force to a required height as if the object does not have any weight.

There is a sensor housing (17) which measures the load (force) carried by the user, is positioned with two vertical bearings (18, 19) positioned on the carrier slot (8) within the strength control system. The force sensors (23) in this housing (17) measure the load coming to the system at this point in a precise manner. The microprocessor receives the information coming from these sensors (23), determines the counter balancing force required by the system and sends the motion command to the electric motor (13) according to the size of this force.

The electric motor (13) is connected to a ball screw (15). There are two reverse gear bearings (16) on this shaft (15). These bearings (16) move towards or opposite to each other with the movement of the shaft in one direction. This movement moves one end of the brackets (10) backwards and forwards. Since the other end of the bracket (10) is connected to the gas piston (9), this forward-backward motion moves one end of the gas piston (9) away from the vertical bearing axis. The angle of the gas piston (9) formed with the vertical axis and the piston length changes because the other end of the gas piston (9) is connected to the vertical bearing (18).

There is a rechargeable or disposable battery which supplies the energy required by electric motors (13), sensors (23) and microprocessor in the system.

In the direction of user demand, the angular position of the gas pistons can be fixed by means of using an adjustment arm rather than the electric motor (13) for additionally reducing the costs and weight. In this case, prior to use, each time the user wears the system by making manual adjustments and gets a constant force support at a rate determined by the adjustment arm from the system.

### Industrial Applicability of the Invention

The invention is suitable for people who work in many fields such as military, agriculture, industry and transportation; are obliged to work by lifting compulsory high weights only by manpower, or have to carry loads of variable weight with arm force. Loading of vegetable boxes into the vehicle, cargo workers, assembly operators, and airport loading area employees can be shown as examples to these.

## Claims

1. Wearable strength-supporting system,
• wherein it is fixed to the human body from waist, back and shoulder regions and arms,
• wherein it has carrier arms (7) which form a respective planar pantograph structure for each of the right and left arms, are connected with planar rotary joints having low friction, have metallic or composite structure,
• wherein it has a strength control system which allows the carried load to be moved to the required height without applying any force in the vertical direction such that the object does not have any weight, forms a balancing counter force by detecting the weight of the carried load.

2. Wearable strength supporting system according to claim 1, wherein it has waist support (3), back support (2), side hip support (4) and wrist or biceps supports (6) which are made of leather or fabric on the sections that contact the human body and are filled with sponge or similar soft and flexible structure to reduce pressure on the body on the waist, back, side hips and wrists or biceps as the main contact points, have an outer part made of hard plastic, metal or composite.

3. Wearable strength supporting system according to claim 1, wherein it has a waist belt (5) with adjustable height which enables to transfer the forces coming from the waist support (3) to the front portions of the body.

4. Wearable strength supporting system according to claim 1, wherein it has a metallic or composite body skeleton (1) which connects the waist, back and side hip contact points.

5. Wearable strength supporting system according to claim 1 and claim 4, wherein the body skeleton (1) has rotary joints which enables to transfer the force to the body by rotating at the contact points of the body in the waist (24) and back (25) region.

6. Wearable strength supporting system according to claim 1, wherein it has shoulder straps (11) with adjustable height fixed on both shoulders in order to transfer the weight of the system in the vertical direction to the body.

7. Wearable strength supporting system according to claim 1, wherein it comprises carrier slots (8), one for right arm and one for left arm, which enables the connection of the carrier arms where the horizontal and vertical movement of the carrier arms (7) are controlled, to the body skeleton.

8. Wearable strength supporting system according to claim 1, claim 4 and claim 7, wherein it comprises planar rotary joints (14), one for right arm and one for left arm, which allows the whole system to rotate on the vertical axis, enables the connection of the carrier slot (8) where the horizontal and vertical movement of the carrier arms (7) are controlled, to the body skeleton (1).

9. Wearable strength supporting system according to claim 1, wherein each carrier arm has a low-friction bearing (20) and a horizontal linear slide (21) which enable the user to move the load carried in the horizontal axis without being exposed to any opposing resistance other than friction, have low friction, are linear and are aligned in the horizontal axis.

10. Wearable strength supporting system according to claim 1, wherein each carrier arm has low-friction bearings (18,19) and a vertical linear slide (22) which ensure the user to move the carried load in the vertical direction without applying any force to a required height as if the object does not have any weight, have low friction, are aligned in the vertical axis.

11. Wearable strength supporting system according to claim 1, wherein it has crosswise supported brackets (10) to each other whose one end is connected to gas pistons (9) and the other end is connected to reverse gear ball nuts (16) on a ball screw (15) by means of planar rotary joints, which can be able to change the angle between the gas pistons (9) and the vertical axis when it is driven by an electric motor (13).

12. Wearable strength supporting system according to claim 2, wherein it has a rotary joint (26) which allows the rotational movement of the end point of a carrier arm and a wrist or biceps support (6) relative to each other in the biceps or wrist sections of the arm of the user.

13. Wearable strength supporting system according to claim 10, wherein it has a sensor housing (17) which is located with said two low-friction -bearings (18,19) on said vertical linear slide and force sensors (23) located therein which measure the load (force) that the user carries on the system.

14. Wearable strength supporting system according to claim 11, wherein it has a microprocessor which determines the angle required to be formed by the gas pistons (9) with the vertical axis against the load measured by using the data coming from a force sensor (23) and activates the electric motor (13).

## Patentansprüche

1. Tragbares kraftunterstützendes System,
• wobei es am menschlichen Körper an Taille, Rücken, Schulterbereichen und Armen befestigt ist,
• wobei es Tragarme (7) aufweist, die jeweils eine ebene Pantographenstruktur für jeden der rechten und linken Arme bilden, die mit ebenen Drehgelenken mit geringer Reibung verbunden sind und eine metallische oder Verbundstruktur haben,
• wobei es ein Kraftsteuerungssystem hat, das es ermöglicht, die getragene Last ohne Anwendung einer Kraft in vertikaler Richtung auf die erforderliche Höhe zu bewegen, so dass das Objekt kein Gewicht hat und eine ausgleichende Gegenkraft durch die Erfassung des Gewichts der getragenen Last bildet.

2. Tragbares kraftunterstützendes System gemäß Anspruch 1, wobei es eine Taillenstütze (3), eine Rückenstütze (2), eine seitliche Hüftstütze (4) und Handgelenk- oder Bizepsstützen (6) aufweist, die aus Leder oder Stoff in den Abschnitten gefertigt sind, die den menschlichen Körper berühren und mit Schwamm oder einer ähnlichen weichen und flexiblen Struktur gefüllt sind, um den Druck auf den Körper an Taille, Rücken, seitlichen Hüften sowie Handgelenken oder Bizepsen als Hauptkontaktpunkte zu reduzieren, und eine Außenseite aus hartem Kunststoff, Metall oder Verbundwerkstoff haben.

3. Tragbares kraftunterstützendes System gemäß Anspruch 1, wobei es einen Taillengurt (5) mit verstellbarer Höhe aufweist, der die Übertragung der Kräfte von der Taillenstütze (3) auf die vorderen Teile des Körpers ermöglicht.

4. Tragbares kraftunterstützendes System gemäß Anspruch 1, wobei es ein metallisches oder Verbundkörper-Skelett (1) aufweist, das die Kontaktpunkte an Taille, Rücken und seitlichen Hüften verbindet.

5. Tragbares kraftunterstützendes System gemäß Anspruch 1 und Anspruch 4, wobei das Körperskelett (1) Drehgelenke aufweist, die es ermöglichen, die Kraft durch Drehen an den Kontaktpunkten des Körpers im Bereich der Taille (24) und des Rückens (25) auf den Körper zu übertragen.

6. Tragbares kraftunterstützendes System gemäß Anspruch 1, wobei es Schultergurte (11) mit verstellbarer Höhe aufweist, die an beiden Schultern befestigt sind, um das Gewicht des Systems in vertikaler Richtung auf den Körper zu übertragen.

7. Tragbares kraftunterstützendes System gemäß Anspruch 1, wobei es Tragschlitze (8) umfasst, einen für den rechten Arm und einen für den linken Arm, die die Verbindung der Tragarme ermöglichen, wobei die horizontalen und vertikalen Bewegungen der Tragarme (7) gesteuert werden, mit dem Körperskelett.

8. Tragbares kraftunterstützendes System gemäß den Ansprüchen 1, 4 und 7, wobei es ebene Drehgelenke (14) umfasst, eines für den rechten Arm und eines für den linken Arm, die es dem gesamten System ermöglichen, um die vertikale Achse zu rotieren, und die Verbindung des Tragschlitzes (8), in dem die horizontalen und vertikalen Bewegungen der Tragarme (7) gesteuert werden, mit dem Körperskelett (1) ermöglichen.

9. Tragbares kraftunterstützendes System gemäß Anspruch 1, wobei jeder Tragarm ein reibungsarmes Lager (20) und eine horizontale Linearschiene (21) aufweist, die es dem Benutzer ermöglichen, die getragene Last entlang der horizontalen Achse zu bewegen, ohne einer anderen Widerstandskraft als der Reibung ausgesetzt zu sein, wobei sie eine geringe Reibung aufweisen, linear sind und entlang der horizontalen Achse ausgerichtet sind.

10. Tragbares kraftunterstützendes System gemäß Anspruch 1, wobei jeder Tragarm reibungsarme Lager (18,19) und eine vertikale Linearschiene (22) aufweist, die es dem Benutzer ermöglichen, die getragene Last in vertikaler Richtung auf die erforderliche Höhe zu bewegen, ohne eine Kraft aufzuwenden, sodass das Objekt kein Gewicht zu haben scheint, wobei sie eine geringe Reibung aufweisen und entlang der vertikalen Achse ausgerichtet sind.

11. Tragbares kraftunterstützendes System gemäß Anspruch 1, wobei es sich kreuzweise unterstützende Halterungen (10) aufweist, deren ein Ende mit Gaszylindern (9) verbunden ist und deren anderes Ende über ebene Drehgelenke mit Rückwärtsgang-Kugelmuttern (16) auf einer Kugelspindel verbunden ist, wobei der Winkel zwischen den Gaszylindern (9) und der vertikalen Achse verändert werden kann, wenn sie von einem Elektromotor (13) angetrieben werden.

12. Tragbares kraftunterstützendes System gemäß Anspruch 2, wobei es ein Drehgelenk (26) aufweist, das die Drehbewegung des Endpunkts eines Tragarms und einer Handgelenk- oder Bizepsstütze (6) relativ zueinander in den Bereichen des Bizeps oder des Handgelenks des Arms des Benutzers ermöglicht.

13. Tragbares kraftunterstützendes System gemäß Anspruch 10, wobei es ein Sensorgehäuse (17) aufweist, das zusammen mit den beiden reibungsarmen Lagern (18,19) auf der vertikalen Linearschiene (22) angeordnet ist, und darin befindliche Kraftsensoren (23), die die Last (Kraft) messen, die der Benutzer mit dem System trägt.

14. Tragbares kraftunterstützendes System gemäß Anspruch 11, wobei es einen Mikroprozessor aufweist, der den Winkel bestimmt, den die Gaszylinder (9) mit der vertikalen Achse in Abhängigkeit von der durch einen Kraftsensor (23) gemessenen Last einnehmen müssen, und der den Elektromotor (13) aktiviert.

## Revendications

1. Système de support de force portable,
• où il est fixé au corps humain au niveau de la taille, du dos, des épaules et des bras,
• où il possède des bras porteurs (7) formant une structure de pantographe planaire respective pour chacun des bras droit et gauche, sont connectés avec des articulations rotatives planes ayant une faible friction, ont une structure métallique ou composite,
• où il dispose d'un système de contrôle de la force qui permet de déplacer la charge transportée à la hauteur requise sans appliquer de force dans la direction verticale de sorte que l'objet n'ait aucun poids, forme une contre-force d'équilibrage en détectant le poids de la charge transportée.

2. Système de support de force portable selon la revendication 1, où il comprend un support de taille (3), un support dorsal (2), un support de hanche latéral (4) et des supports de poignet ou de biceps (6) qui sont fabriqués en cuir ou en tissu sur les sections en contact avec le corps humain et sont remplis d'éponge ou d'une structure similaire souple et flexible pour réduire la pression sur le corps au niveau de la taille, du dos, des hanches latérales et des poignets ou des biceps comme principaux points de contact, ont une partie extérieure faite de plastique dur, de métal ou de composite.

3. Système de support de force portable selon la revendication 1, où il comprend une ceinture de taille (5) à hauteur réglable qui permet de transférer les forces provenant du support de taille (3) vers les parties avant du corps.

4. Système de support de force portable selon la revendication 1, où il comprend un squelette de corps métallique ou composite (1) qui relie les points de contact de la taille, du dos et des hanches latérales.

5. Système de support de force portable selon les revendications 1 et 4, où le squelette de corps (1) possède des articulations rotatives qui permettent de transférer la force au corps en pivotant aux points de contact du corps dans la région de la taille (24) et du dos (25).

6. Système de support de force portable selon la revendication 1, où il comprend des bretelles (11) à hauteur réglable fixées sur les deux épaules afin de transférer le poids du système dans la direction verticale vers le corps.

7. Système de support de force portable selon la revendication 1, où il comprend des fentes de port (8), une pour le bras droit et une pour le bras gauche, qui permettent la connexion des bras porteurs où les mouvements horizontaux et verticaux des bras porteurs (7) sont contrôlés, au squelette du corps.

8. Système de support de force portable selon les revendications 1, 4 et 7, où il comprend des articulations rotatives planes (14), une pour le bras droit et une pour le bras gauche, qui permettent à l'ensemble du système de pivoter sur l'axe vertical, facilitent la connexion de la fente de port (8) où les mouvements horizontaux et verticaux des bras porteurs (7) sont contrôlés, au squelette du corps (1).

9. Système de support de force portable selon la revendication 1, où chaque bras porteur possède un palier à faible friction (20) et un coulisseau linéaire horizontal (21) qui permettent à l'utilisateur de déplacer la charge portée dans l'axe horizontal sans être exposé à une résistance opposée autre que la friction, ont une faible friction, sont linéaires et sont alignés dans l'axe horizontal.

10. Système de support de force portable selon la revendication 1, où chaque bras porteur possède des roulements à faible friction (18,19) et un coulisseau linéaire vertical (22) qui permettent à l'utilisateur de déplacer la charge portée dans la direction verticale sans appliquer de force à une hauteur requise comme si l'objet n'avait aucun poids, ont une faible friction, sont alignés dans l'axe vertical.

11. Système de support de force portable selon la revendication 1, où il comprend des supports croisés (10) reliés l'un à l'autre dont une extrémité est connectée à des pistons à gaz (9) et l'autre extrémité est connectée à des écrous à billes de marche arrière (16) sur une vis à billes par des articulations rotatives planes, qui peuvent changer l'angle entre les pistons à gaz (9) et l'axe vertical lorsqu'ils sont actionnés par un moteur électrique (13).

12. Système de support de force portable selon la revendication 2, où il comprend une articulation rotative (26) qui permet le mouvement rotatif du point d'extrémité d'un bras porteur et d'un support de poignet ou de biceps (6) l'un par rapport à l'autre dans les sections du biceps ou du poignet du bras de l'utilisateur.

13. Système de support de force portable selon la revendication 10, où il comprend un logement de capteur (17) situé avec les deux roulements à faible friction (18,19) sur le coulisseau linéaire vertical (22) mentionné, et des capteurs de force (23) y étant placés qui mesurent la charge (force) que l'utilisateur porte sur le système.

14. Système de support de force portable selon la revendication 11, où il comprend un microprocesseur qui détermine l'angle requis à former par les pistons à gaz (9) avec l'axe vertical en fonction de la charge mesurée en utilisant les données provenant d'un capteur de force (23) et active le moteur électrique (13).
